# EUROPÄISCHE PATENTSCHRIFT

(11) **EP 2 203 198 B1**
(45) Veröffentlichungstag und Bekanntmachung des Hinweises auf die Patenterteilung: **25.01.2017**
(21) Anmeldenummer: 08845413.7
(22) Anmeldetag: 31.10.2008
(51) Int. Cl.: A61M 1/36, A61M 1/34

(54) **VERFAHREN UND VORRICHTUNG ZUR ÜBERWACHUNG DER ZUFUHR VON SUBSTITUTIONSFLÜSSIGKEIT WÄHREND EINER EXTRAKORPORALEN BLUTBEHANDLUNG**
METHOD AND APPARATUS FOR MONITORING THE SUPPLY OF REPLACEMENT FLUID DURING AN EXTRACORPOREAL TREATMENT OF BLOOD
PROCÉDÉ ET DISPOSITIF DE SURVEILLANCE DE L'AMENÉE D'UN LIQUIDE DE SUBSTITUTION PENDANT UN TRAITEMENT EXTRACORPOREL DU SANG

(30) Priorität: 03.11.2007 DE 102007052571
(43) Veröffentlichungstag der Anmeldung: 07.07.2010
(73) Patentinhaber: Fresenius Medical Care Deutschland GmbH, 61352 Bad Homburg v.d.H. (DE)
(72) Erfinder: NOACK, Joachim, 97616 Bad Neustadt (DE); DREYHSIG, Jörg, 66606 St. Wendel (DE)
(74) Vertreter: Oppermann, Frank
(86) Internationale Anmeldenummer: PCT/EP2008/009192
(87) Internationale Veröffentlichungsnummer: WO 2009/056325

(56) Entgegenhaltungen:
- EP-A- 0 089 003
- EP-A- 1 175 917
- WO-A-03/047656

## Beschreibung

Die Erfindung betrifft eine Vorrichtung zur Überwachung der Zufuhr von Substitutionsflüssigkeit für eine Vorrichtung zur extrakorporalen Blutbehandlung sowie eine extrakorporale Blutbehandlungsvorrichtung mit einer Überwachungsvorrichtung für die Zufuhr von Substitutionsflüssigkeit.

Zur Entfernung von harnpflichtigen Substanzen und zum Flüssigkeitsentzug werden verschiedene Verfahren zur extrakorporalen Blutbehandlung bzw. -reinigung eingesetzt. Bei der Hämodialyse wird das Blut des Patienten außerhalb des Körpers in einem Dialysator gereinigt. Der Dialysator weist eine Blutkammer und eine Dialysierflüssigkeitskammer auf, die von einer semipermeablen Membran getrennt sind. Während der Behandlung strömt Blut des Patienten durch die Blutkammer. Um das Blut effektiv von harnpflichtigen Substanzen zu reinigen, wird die Dialysierflüssigkeitskammer kontinuierlich von frischer Dialysierflüssigkeit durchströmt.

Während bei der Hämodialyse (HD) der Transport der kleineren molekularen Substanzen durch die Membran des Dialysators im Wesentlichen durch die Konzentrationsunterschiede (Diffusion) zwischen der Dialysierflüssigkeit und dem Blut bestimmt wird, werden bei der Hämofiltration (HF) im Plasmawasser gelöste Substanzen, insbesondere höhermolekulare Stoffe, durch einen hohen Flüssigkeitsstrom (Konvektion) durch die Membran des Dialysators effektiv entfernt. Bei der Hämofiltration fungiert der Dialysator als Filter. Eine Kombination aus beiden Verfahren ist die Hämodiafiltration (HDF).

Bei der Hämo(dia)filtration wird ein Teil des durch die Membran des Dialysators abgezogenen Serums durch eine sterile Substitutionsflüssigkeit ersetzt, die im allgemeinen entweder stromauf des Dialysators oder stromab des Dialysators dem extrakorporalen Blutkreislauf zugeführt wird. Die Zufuhr der Substitutionsflüssigkeit stromauf des Dialysators wird auch als Prädilution und die Zufuhr stromab des Dialysators als Postdilution bezeichnet.

Es sind Vorrichtungen zur Hämo(dia)filtration bekannt, bei denen die Dialysierflüssigkeit online aus Frischwasser und Dialysierflüssigkeitskonzentrat und die Substitutionsflüssigkeit online aus der Dialysierflüssigkeit hergestellt werden.

Bei den bekannten Hämo(dia)filtrationsvorrichtungen wird die Substitutionsflüssigkeit (Substituat) dem extrakorporalen Blutkreislauf von dem Flüssigkeitssystem der Maschine über eine Substituatzuführleitung zugeführt. Bei der Prädilution führt die Substituatleitung zu einer Anschlussstelle an der arteriellen Blutleitung stromauf des Dialysators oder Filters, während bei der Postdilution die Substituatleitung zu einer Anschlussstelle an der venösen Blutleitung stromab des Dialysators oder Filters führt. Die Substituatleitung weist beispielsweise einen Konnektor auf, mit dem sie entweder an die venöse oder arterielle Blutleitung angeschlossen werden kann. Zum Unterbrechen der Flüssigkeitszufuhr ist an der Substituatleitung eine Klemme oder dgl. vorgesehen. Ein derartiges Hämo(dia)filtrationsgerät ist beispielsweise aus der EP-A-0 189 561 bekannt.

Die Effektivität der Blutbehandlung hängt davon ab, ob die Substitutionsflüssigkeit stromauf oder stromab des Dialysators oder Filters dem extrakorporalen Blutkreislauf zugeführt wird. Daher ist die Kenntnis des Behandlungsmodus, d.h. ob eine Prä- oder Postdilution erfolgt, von Bedeutung.

Die EP 0 089 003 A2 beschreibt eine Blutbehandlungsvorrichtung, die über eine Ultrafiltrationseinrichtung und eine Einrichtung zur Überwachung des Blutvolumens verfügt. Darüber hinaus weist die Blutbehandlungsvorrichtung eine Steuereinheit und eine Auswerteinheit auf, um in Abhängigkeit von dem Blutvolumen des Patienten die Ultrafiltrationseinrichtung unter Berücksichtigung eines vorgegebenen Profils zu steuern.

Eine Blutbehandlungsvorrichtung, die über eine Einrichtung für die Zufuhr von Substitutionsflüssigkeit stromauf oder stromab des Dialysators verfügt, ist aus der EP 1 175 917 A1 bekannt. In Abhängigkeit von bestimmten Parametern der Dialyse wird die Zufuhr von Substituat stromauf oder stromab des Dialysators geregelt.

Die EP-A-1 348 458 A1 beschreibt ein Verfahren und eine Vorrichtung zum Überwachen der Zufuhr von Substitutionsflüssigkeit für eine extrakorporale

Blutbehandlungsvorrichtung. Für die Detektion der Zufuhr von Substitutionsflüssigkeit stromauf oder stromab des Dialysators oder Filters wird die Laufzeit der Druckwellen einer in der Substituatleitung angeordneten Substituatpumpe gemessen. Die Zufuhr von Substituat stromauf oder stromab des Dialysators oder Filters wird auf der Grundlage der Laufzeitmessung erkannt. Das bekannte Verfahren setzt den Einsatz einer Druckwellen erzeugenden Substituatpumpe voraus.

Aus der DE 10 2004 023 080 A1 ist eine Vorrichtung zur Überwachung der Zufuhr von Substitutionsflüssigkeit bekannt, bei der die Zufuhr von Substituat stromauf oder stromab des Dialysators oder Filters auf der Grundlage der Veränderung des Drucks, beispielsweise auf Grund eines plötzlichen Druckanstiegs und/oder Druckabfalls nach dem Abschalten oder Einschalten der Substituatpumpe erkannt wird. Die bekannte Vorrichtung setzt den Einsatz einer Druckwellen erzeugenden Substituatpumpe nicht voraus.

Der Erfindung liegt die Aufgabe zu Grunde, eine Vorrichtung zur Überwachung der Zufuhr von Substitutionsflüssigkeit anzugeben, mit der Prä- und Postdilution zuverlässig erkannt werden kann. Eine weitere Aufgabe der Erfindung ist, eine extrakorporale Blutbehandlungsvorrichtung mit einer derartigen Überwachungsvorrichtung zu schaffen.

Die Lösung dieser Aufgaben erfolgt erfindungsgemäß mit den in den unabhängigen Patentansprüchen angegebenen Merkmalen. Bevorzugte Ausführungsformen der Erfindung sind Gegenstand der Unteransprüche.

Die erfindungsgemäße Vorrichtung zur Erkennung von Prä- bzw. Postdilution beruht auf der Messung und Überwachung der Dichte des Bluts oder eines Blutinhaltsstoffes im extrakorporalen Kreislauf. Bei einer Veränderung der Substitutionsrate Q_{S}, mit der dem Blut im extrakorporalen Kreislauf Substitutionsflüssigkeit zugeführt wird, und/oder der Blutflussrate Q_{B}, mit der Blut der ersten Kammer des Dialysators oder Filters zugeführt wird und/oder der Flussrate Q_{M}, mit der Flüssigkeit dem Blut über die Membran des Dialysators oder Filters entzogen wird, verändert sich die Dichte des Bluts oder des Blutinhaltsstoffes im extrakorporalen Blutkreislauf. Es hat sich gezeigt, dass der Betrag und/oder die Richtung der Veränderung, d.h. eine Erhöhung oder Verringerung der Dichte um einen bestimmten Wert, davon abhängig ist, ob die Substitutionsflüssigkeit dem Blut stromauf oder stromab des Dialysators oder Filters zugeführt wird. Auf eine Prädilution oder Postdilution wird dann auf der Grundlage der Veränderung der Dichte des Bluts oder des Blutinhaltsstoffes geschlossen. Als Dichteänderung ist in diesem Sinn auch die Konzentrationsänderung eines Blutinhaltsstoffes wie z.B. Hämoglobin zu verstehen.

Die erfindungsgemäße Vorrichtung setzt grundsätzlich nur die einmalige Veränderung der Substitutionsrate Q_{S} und/oder Blutflussrate Q_{B} und/oder der Flussrate Q_{M} voraus, mit der Flüssigkeit dem Blut über die Membran des Dialysators oder Filters entzogen wird. Da die Prä- oder Postdilution während der Blutbehandlung überwacht werden soll, die vorzugsweise mit bestimmten Flüssigkeitsraten Q_{S}, Q_{B} und/oder Q_{M} durchgeführt werden soll, sieht eine bevorzugte Ausführungsform nach der Verringerung bzw. Erhöhung mindestens einer der drei Flüssigkeitsraten um einen vorgegebenen Betrag für ein vorgegebenes Zeitintervall, das möglichst klein sein sollte, wieder eine Erhöhung bzw. Verringerung nach Ablauf des vorgegebenen Zeitintervalls um einen vorgegebenen Betrag vor, der insbesondere dem Betrag entspricht, um den die entsprechende Flüssigkeitsrate oder die Flüssigkeitsraten zuvor verringert bzw. erhöht worden ist bzw. sind, so dass die Blutbehandlung mit den gleichen Flüssigkeitsraten weitergeführt werden kann. Dabei wird die dem Blut entzogene Flussrate Q_{M} vorzugsweise gleichzeitig in dem gleichen Zeitintervall vorzugsweise um den gleichen Betrag verringert bzw. erhöht und nach Ablauf des vorgegebenen Zeitintervalls vorzugsweise um den gleichen Betrag wieder erhöht bzw. verringert, wie die Substitutionsrate Q_{S}.

Wenn nachfolgend von einer Veränderung der Flussrate die Rede ist, kann darunter auch eine Verringerung der Flussrate um einen Betrag verstanden werden, so dass die Flussrate Null ist, d.h. der Fluss unterbrochen ist.

Für die Detektion von Prä- bzw. Postdilution ist grundsätzlich unerheblich, um welchen Betrag eine Flussrate verringert oder vergrößert wird. Entscheidend ist aber, dass eine Veränderung der Dichte mit ausreichender Sicherheit selektiv für die Prä- und die Postdilution nachgewiesen werden kann.

Die erfindungsgemäße Vorrichtung sieht unterschiedliche Ausführungsformen vor, die sich dadurch unterscheiden, an welcher Stelle des extrakorporalen Blutkreislaufs die Dichte des Bluts oder des Blutinhaltsstoffes gemessen wird.

Eine Prä- oder Postdilution kann durch eine Messung der Dichte stromab der Stelle des extrakorporalen Blutkreislaufs, an der dem Blutkreislauf bei einer Prädilution Substitutionsflüssigkeit zugeführt wird, und stromauf der ersten Kammer des Dialysators oder Filters erkannt werden. Es ist auch möglich, eine Prä- oder Postdilution durch eine Messung der Dichte stromab der ersten Kammer des Dialysators oder Filters und stromauf der Stelle des Blutkreislaufs, an der dem Blutkreislauf bei einer Postdilution Substitutionsflüssigkeit zugeführt wird, zu detektieren. Die Dichte kann auch durch eine Messung stromab der Stelle des Blutkreislaufs nachgewiesen werden, an der dem Blutkreislauf bei einer Postdilution Substitutionsflüssigkeit zugeführt wird. Es kann entscheidend sein, dass die Dichte unmittelbar nach der Veränderung der jeweiligen Flussrate gemessen wird, da je nach Messposition eine Änderung der Dichte nur innerhalb eines bestimmten Zeitintervalls nachgewiesen werden kann. Denn nach Ablauf dieses Zeitintervalls kann die Dichte in diesen Fällen wieder ihren ursprünglichen Wert annehmen.

Es hat sich gezeigt, dass die Veränderung der Substitutionsrate Q_{S} bei gleichzeitiger Veränderung der Flussrate Q_{M}, mit der Flüssigkeit dem Blut über die Membran des Dialysators oder Filters entzogen wird, an den unterschiedlichen Stellen des extrakorporalen Kreislaufs zu besonders unterschiedlichen Veränderungen der Dichte führt. Beispielsweise kann die Dichte in Abhängigkeit von einer Prä- oder Postdilution zu- oder abnehmen.

Bei einer ersten Ausführungsform wird die Substitutionsrate Q_{S} um einen vorgegebenen Betrag verringert und die Dichte wird im Blutkreislauf stromab der Stelle des Blutkreislaufs, an der dem Blutkreislauf bei einer Prädilution Substitutionsflüssigkeit zugeführt wird, und stromauf der ersten Kammer des Dialysators oder Filters gemessen. Die Dichte des Bluts oder Blutinhaltsstoffes vor der Verringerung der Substitutionsrate Q_{S} und nach der Verringerung der Substitutionsrate Q_{S} werden dann miteinander verglichen, wobei auf eine Zufuhr von Substitutionsflüssigkeit stromauf des Dialysators oder Filters geschlossen wird, wenn die Dichte nach der Verringerung der Substitutionsrate um einen vorgegebenen Betrag zugenommen hat. Wenn die Dichte des Bluts nach der Verringerung der Substitutionsrate nicht um einen vorgegebenen Betrag zugenommen hat, wird hingegen auf eine Zufuhr von Substitutionsflüssigkeit stromab des Dialysators oder Filters geschlossen.

Die erfindungsgemäße Vorrichtung setzt grundsätzlich nicht die Messung der Dichte des Bluts sowohl vor als auch nach der Verringerung der Substitutionsrate voraus. Es kann grundsätzlich ausreichend sein, die Dichte nur nach der Verringerung der Substitutionsrate zu messen, um den gemessenen Wert mit einem charakteristischen Grenzwert zu vergleichen.

Eine besonders bevorzugte Ausführungsform mit einer besonders signifikanten Änderung der Dichte des Bluts sieht eine Messung der Dichte des Bluts oder Blutinhaltsstoffe im Blutkreislauf stromab der Stelle des Blutkreislaufs vor, an der dem Blutkreislauf bei einer Postdilution Substitutionsflüssigkeit zugeführt wird. Auf eine Zufuhr von Substitutionsflüssigkeit stromauf des Dialysators oder Filters wird nach einem Vergleich der Dichte vor und nach der Verringerung der Substitutionsrate Q_{S} und bevorzugt einer gleichzeitigen Verringerung der Flussrate Q_{M} geschlossen, wenn die Dichte nach der Verringerung der Substitutionsrate Q_{S} um einen vorgegebenen Betrag abgenommen hat. Auf eine Zufuhr von Substitutionsflüssigkeit stromab des Dialysators oder Filters wird geschlossen, wenn die Dichte nach der Verringerung der Substitutionsrate Q_{S} um einen vorgegebenen Betrag zugenommen hat.

Die Zunahme der Dichte des Bluts oder des Blutinhaltsstoffes bei der Postdilution ist darauf zurückzuführen, dass unmittelbar nach der Verringerung der Substitutionsrate Q_{S}, mit der Substitutionsflüssigkeit dem Blut zugeführt wird, und der gleichzeitigen Verringerung der Flüssigkeitsrate Q_{M}, mit der dem Blut über die Membran des Dialysators oder Filters Flüssigkeit entzogen wird, dem nunmehr aus dem Dialysator strömenden Blut über die Membran noch eine entsprechende Menge an Flüssigkeit entzogen worden ist. Dadurch ist das aus dem Dialysator oder Filter fließende Blut unmittelbar nach der Verringerung der Raten Q_{S} und Q_{M} eingedickt. Eine Verringerung der Menge der dem Blut nach dem Durchtritt durch den Dialysator zugeführten Substitutionsflüssigkeit (Postdilution) führt daher unmittelbar zu einem Anstieg der Dichte des Bluts oder des Blutinhaltsstoffes im Blutkreislauf stromab des Dialysators. Wenn bei einer Prädilution die Raten Q_{S} und Q_{M} hingegen verringert werden, ist das im Dialysator oder Filter befindliche Blut bereits durch den vorherigen Zufluss von Substitutionsflüssigkeit verdünnt worden. Da die dem Substituatfluss entsprechende Filtration im Dialysator verringert wird oder ausbleibt, nimmt die Dichte des zum Patienten zurückzuströmenden Bluts ab. Auf eine Zufuhr von Substitutionsflüssigkeit stromauf des Dialysators oder Filters (Prädilution) wird also dann geschlossen, wenn die Dichte des Bluts oder des Blutinhaltsstoffes nach der Verringerung der Substitutionsrate Q_{S} um einen vorgegebenen Betrag abgenommen hat oder um einen Betrag abgenommen hat, der größer als der vorgegebene Grenzwert ist.

Folglich kann auf der Grundlage der Zu- bzw. Abnahme der Dichte des Bluts oder des Blutinhaltsstoffes nach der Verringerung der Substitutionsrate Q_{S} auf eine Post- bzw. Prädilution mit hoher Sicherheit geschlossen werden. Dabei sollte aber die Zu- bzw. Abnahme der Dichte einen vorgegebenen Grenzwert überschreiten, um sicher zwischen einer Veränderung der Dichte des Bluts auf Grund einer Veränderung der Flussraten und allgemeinen Dichteschwankungen des Bluts unterscheiden zu können.

Anstelle einer Verringerung der Substitutionsrate Q_{S} ist auch eine Erhöhung der Substitutionsrate Q_{S} möglich, mit der dem Blut Flüssigkeit entzogen wird. Bei dieser Ausführungsform kann die Dichte stromab der Stelle des Blutkreislaufs, an der dem Blutkreislauf bei einer Prädilution Substitutionsflüssigkeit zugeführt wird, und stromauf der ersten Kammer des Dialysators oder Filters gemessen werden. Dann wird auf eine Zufuhr von Substitutionsflüssigkeit stromauf des Dialysators (Prädilution) oder Filters geschlossen, wenn die Dichte nach der Vergrößerung der Substitutionsrate um einen vorgegebenen Betrag abgenommen hat, und auf eine Zufuhr von Substitutionsflüssigkeit stromab des Dialysators oder Filters (Postdilution) geschlossen, wenn die Dichte nach der Vergrößerung der Substitutionsrate nicht um einen vorgegebenen Betrag abgenommen hat.

Alternativ kann bei dieser Ausführungsform die Dichte des Bluts oder des Blutinhaltsstoffes im Blutkreislauf auch stromab der Stelle des Blutkreislaufs gemessen werden, an der dem Blutkreislauf bei einer Postdilution Substitutionsflüssigkeit zugeführt wird. Dann kann auf eine Zufuhr von Substitutionsflüssigkeit stromauf des Dialysators oder Filters bevorzugt bei einer gleichzeitigen Erhöhung der Flussrate Q_{M} geschlossen werden, wenn die Dichte nach der Vergrößerung der Substitutionsrate Q_{S} um einen vorgegebenen Betrag zugenommen hat, und auf eine Zufuhr von Substitutionsflüssigkeit stromab des Dialysators oder Filters geschlossen werden, wenn die Dichte nach der Verringerung der Substitutionsrate Q_{S} um einen vorgegebenen Betrag abgenommen hat.

Als Dichte des Bluts kann sowohl die physikalische Dichte oder Massendichte, die eine Massenverteilung beschreibt, als auch die optische Dichte des Bluts gemessen werden, die ein Maß für die Abschwächung einer Strahlung (beispielsweise Licht) in einem Medium, d.h. des Bluts ist.

Es kommen insbesondere alle Messverfahren in Frage, mit denen eine Messung der physikalischen oder optischen Dichte des Bluts oder eines seiner Inhaltsstoffe möglich ist. Eine erste Ausführungsform sieht für die Messung der Änderung der Dichte eine Messung der Ausbreitungsgeschwindigkeit von Ultraschall im Blut entlang einer Messstrecke vor, während eine alternative Ausführungsform die Messung der Dämpfung von Licht im Blut entlang einer Messstrecke vorsieht. Die hierfür erforderlichen Messeinrichtungen sind dem Fachmann allgemein bekannt. Im Übrigen sind optische Detektoren zur Erkennung von Blut und Ultraschallmessstrecken zur Erkennung von Luft in den bekannten Dialysegeräten im Allgemeinen ohnehin vorhanden.

Bei einer weiteren besonders bevorzugten Ausführungsform wird bei der Erkennung einer Prädilution ein den Betriebszustand der Prädilution signalisierendes Signal erzeugt, während bei der Erkennung einer Postdilution ein den Betriebszustand der Postdilution signalisierendes Signal erzeugt wird. Dieses Signal für die Prä- bzw. Postdilution kann weitere in der Blutbehandlungsvorrichtung vorgesehene Einrichtungen steuern. Beispielsweise kann ein Eingriff in die Blutbehandlung vorgenommen werden.

Die erfindungsgemäße Vorrichtung zur Überwachung der Zufuhr von Substitutionsflüssigkeit kann Bestandteil einer Blutbehandlungsvorrichtung sein oder eine getrennte Baugruppe bilden. Da die für die Überwachungsvorrichtung erforderlichen Komponenten im Allgemeinen in den bekannten Blutbehandlungsvorrichtungen ohnehin vorhanden sind, bietet sich eine Integration in die Blutbehandlungsvorrichtung an. Beispielsweise kann auf die entsprechenden Sensoren zur Dichtemessung zurückgegriffen werden. Auch steht eine Mikroprozessorsteuerung zur Verfügung. Damit ist der apparative Aufwand gering.

Die erfindungsgemäße Überwachungsvorrichtung verfügt über eine Steuereinheit zum Steuern der Substitutionseinrichtung und der Ultrafiltrationseinrichtung zum Entziehen von Ultrafiltrat über die Dialysatormembran derart, dass die entsprechenden Flussraten Q_{S} und Q_{M} für die Messung eingestellt werden können. Eine Messeinheit dient zum Messen der Dichte des Bluts oder des Blutinhaltsstoffes und eine Auswerteinheit für die Erkennung von Prä- bzw. Postdilution auf der Grundlage der Dichtemessung.

Die erfindungsgemäße Vorrichtung kann dem Anwender nicht nur einen Hinweis auf die Art der Behandlung, d.h. Prä- bzw. Postdilution geben, sondern auch Abweichungen zwischen der tatsächlichen und der gewünschten Behandlungsart erkennen. Darüber hinaus ist eine automatische Dokumentation oder eine automatische Begrenzung von Eingabeparametern möglich. Auch können mit dem erfindungsgemäßen Verfahren und der erfindungsgemäßen Vorrichtung weitere Betriebsparameter der Blutbehandlungsvorrichtung in Abhängigkeit von dem jeweiligen Betriebszustand gesteuert werden.

Auf die zeitliche Dauer der Dichteänderung hat nicht nur die Veränderung der entsprechenden Flussraten, sondern auch weitere Parameter einen Einfluss. Beispielsweise haben das Volumen der Blutkammer des Dialysators und das Volumen der sich an die Blutkammer anschließenden Schlauchleitungsabschnitte einen Einfluss auf die Dauer der Dichteänderung. Insofern kann mit der Dichtemessung auch auf das Volumen eines Teilabschnitts des extrakorporalen Blutkreislaufs geschlossen werden. Auch die Stärke der Dichteänderung hängt von dem eingeschlossenen Blutvolumen ab.

Im Folgenden werden Ausführungsbeispiele der erfindungsgemäßen Blutbehandlungsvorrichtung unter Bezugnahme auf die Figuren im Einzelnen beschrieben.

Es zeigen:
- Fig. 1: eine extrakorporale Blutbehandlungsvorrichtung mit einer Vorrichtung zur Überwachung der Zufuhr von Substitutionsflüssigkeit, insbesondere zur Erkennung von Prä- und Postdilution, in stark vereinfachter schematischer Darstellung,
- Fig. 2A und 2B: den zeitlichen Verlauf der Dichte für den Fall der Prädilution und Postdilution bei einer Verringerung der Substitutionsrate Q_{S} und der Flussrate Q_{M}, mit der dem Blut über die Membran des Dialysators oder Filters Flüssigkeit entzogen wird, um den gleichen Betrag,
- Fig. 3A und 3B: den zeitlichen Verlauf der Dichte für den Fall der Prädilution und Postdilution bei einer Erhöhung der Substitutionsrate Q_{S} und der Flussrate Q_{M}, mit der dem Blut über die Membran des Dialysators oder Filters Flüssigkeit entzogen wird, um den gleichen Betrag, und
- Fig. 4A und 4B: den zeitlichen Verlauf der Dichte für den Fall der Prädilution und Postdilution bei einer Erhöhung der Blutflussrate.

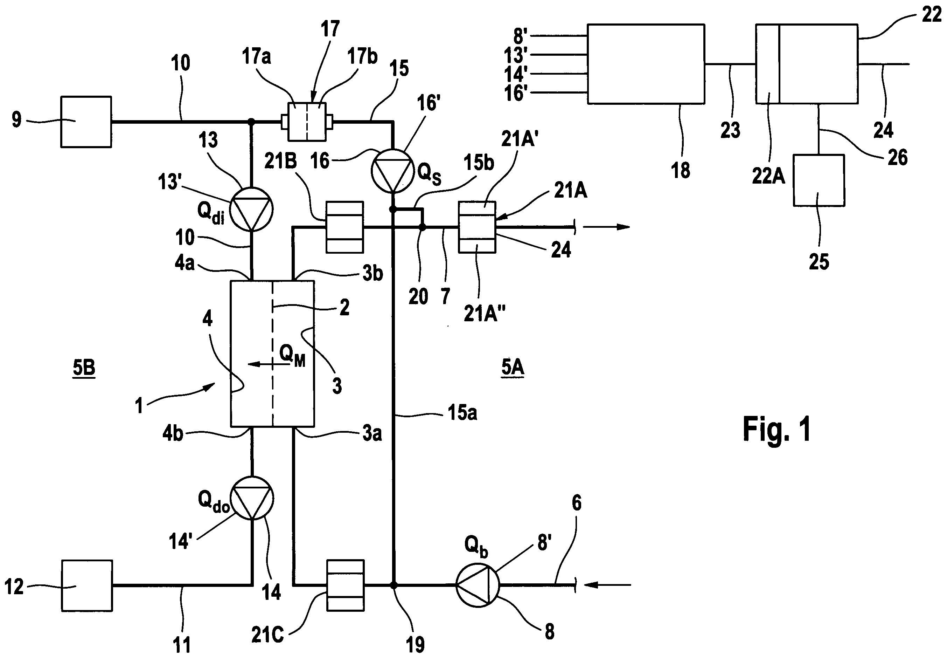

Fig. 1 zeigt nur die wesentlichen Komponenten einer Blutbehandlungsvorrichtung in schematischer Darstellung, die für die Überwachung der Prä- oder Postdilution relevant sind. Bei der vorliegenden Blutbehandlungsvorrichtung handelt es sich um eine Hämo(dia)filtrationsvorrichtung, die einen Dialysator 1 aufweist, der durch eine semipermeable Membran 2 in eine von Blut durchflossene erste Kammer 3, die nachfolgend als Blutkammer bezeichnet wird und eine von Dialysierflüssigkeit durchflossene zweite Kammer 4 getrennt ist, die als Dialysierflüssigkeitskammer bezeichnet wird. Die erste Kammer 3 ist in einen extrakorporalen Blutkreislauf 5A geschaltet, während die zweite Kammer 4 in das Dialysierflüssigkeitssystem 5B der Hämo(dia)filtrationsvorrichtung geschaltet ist.

Der extrakorporale Blutkreislauf 5A umfasst eine arterielle Blutleitung 6, die zu dem Einlass 3a der Blutkammer 3 führt, und eine venöse Blutleitung 7, die von dem Auslass 3b der Blutkammer 3 des Dialysators 1 abgeht. Das Blut des Patienten wird durch die Blutkammer 3 des Dialysators 1 mit einer arteriellen Blutpumpe 8, insbesondere Rollenpumpe gefördert, die an der arteriellen Blutleitung 6 angeordnet ist. Die Blutpumpe führt der Blutkammer 3 des Dialysators Blut mit einer bestimmten Blutflussrate Q_{b} zu. Die Blutleitungen 6, 7 und der Dialysator 3 bilden ein zur einmaligen Verwendung bestimmtes Disposable, das für die Dialysebehandlung in die Dialysevorrichtung eingelegt wird. Zur Eliminierung von Luftblasen können in die arterielle und venöse Blutleitung ein Luftabscheider (Tropfkammer) geschaltet sein.

Die frische Dialysierflüssigkeit wird in einer Dialysierflüssigkeitsquelle 9 bereitgestellt. Von der Dialysierflüssigkeitsquelle 9 führt eine Dialysierflüssigkeitszuführleitung 10 zu dem Einlass 4a der Dialysierflüssigkeitskammer 4 des Dialysators 1. Von dem Auslass 4b der Dialysierflüssigkeitskammer 4 führt eine Dialysierflüssigkeitsabführleitung 11 zu einem Abfluss 12. In die Dialysierflüssigkeitszuführleitung 10 ist eine erste Dialysierflüssigkeitspumpe 13 und in die Dialysierflüssigkeitsabführleitung 11 ist eine zweite Dialysierflüssigkeitspumpe 14 geschaltet. Die erste Dialysierflüssigkeitspumpe 13 fördert Dialysierflüssigkeit von der Dialysierflüssigkeitsquelle mit einer bestimmten Dialysierflüssigkeitszuführrate Q_{di} zu dem Einlass 4a der Dialysierflüssigkeitskammer 4, während die zweite Dialysierflüssigkeitspumpe 14 von dem Auslass 4b der Dialysierflüssigkeitskammer 4 Dialysierflüssigkeit mit einer bestimmten Dialysierflüssigkeitsabführrate Q_{do} zu dem Abfluss 12 fördert.

Während der Dialysebehandlung kann Dialysierflüssigkeit aus dem Dialysierflüssigkeitssystem 5B als Substitutionsflüssigkeit über eine Substitutionsflüssigkeitsleitung 15 dem extrakorporalen Blutkreislauf 5A zugeführt werden, die stromauf der ersten Dialysierflüssigkeitspumpe 13 von der Dialysierflüssigkeitszuführleitung 10 abzweigt.

Die Substitutionsflüssigkeitsleitung 15 weist zwei Leitungsabschnitte 15a und 15b auf, von denen der eine Leitungsabschnitt 15a zu der arteriellen Blutleitung 6 und der andere Leitungsabschnitt 15b zu der venösen Blutleitung 7 führt.

Die Substitutionsflüssigkeit wird mittels einer Substituatpumpe 16, insbesondere Rollenpumpe gefördert, in die die Substitutionsflüssigkeitsleitung 15 eingelegt ist. In die Substitutionsflüssigkeitsleitung 15 ist stromauf der Substituatpumpe eine in zwei Kammern 17a, 17b unterteiltes Sterilfilter 17 geschaltet. Die Substituatpumpe zusammen mit den zugehörigen Leitungen und dem Sterilfilter bilden die Substitutionseinrichtung der Dialysevorrichtung. Zum Abklemmen der beiden Leitungsabschnitte 15a, 15b der Substitutionsflüssigkeitsleitung 15 können Absperrorgane, beispielsweise Schlauchklemmen vorgesehen sein, die aber der besseren Übersichtlichkeit halber nicht dargestellt sind.

Die Blutpumpe 8, die erste und zweite Dialysierflüssigkeitspumpe 13 und 14 sowie die Substituatpumpe 16 sind über Steuerleitungen 8', 13', 14' 16' mit einer zentralen Steuer- und Regeleinheit 18 verbunden, von der die Pumpen unter Berücksichtigung der vorgegebenen Behandlungsparameter angesteuert werden.

Zum Betrieb der Hämo(dia)filtrationsvorrichtung als Hämodialysevorrichtung werden die Blutpumpe 8 sowie die erste und zweite Dialysierflüssigkeitspumpe 13 und 14 betrieben, wobei Dialysierflüssigkeit durch die Dialysierflüssigkeitskammer 4 des Dialysators 1 strömt. Zum Betrieb der Hämo(dia)filtrationsvorrichtung als Hämodiafiltrationsvorrichtung wird die Substituatpumpe 16 betrieben, so dass über den Sterilfilter 17 sterile Dialysierflüssigkeit als Substitutionsflüssigkeit wahlweise zu der arteriellen Zugabestelle 19 stromab der Blutpumpe 8 und stromauf der Blutkammer 3 (Prädilution) oder zu der venösen Zugabestelle 20 stromab der Blutkammer (Postdilution) strömt. Es ist aber auch ein Betrieb der Hämo(dia)filtrationsvorrichtung nur als Hämofiltrationsvorrichtung möglich, wenn die erste Dialysierflüssigkeitspumpe 13nicht betrieben wird und somit der Zufluss von Dialysierflüssigkeit in die Dialysierflüssigkeitskammer des Dialysators unterbrochen wird.

Die Vorrichtung zur Überwachung der Zufuhr von Substitutionsflüssigkeit weist eine Steuereinheit auf, die bei dem vorliegenden Ausführungsbeispiel Teil der zentralen Steuer- und Recheneinheit 18 der Blutbehandlungsvorrichtung ist. Darüber hinaus weist die Vorrichtung zur Detektion von Prä- und Postdilution eine Messeinheit 21A zum Messen der Dichte des Bluts oder eines Blutinhaltsstoffes auf, das aus der Blutkammer 3 des Dialysators 2 über die venöse Blutleitung 7 zurück zum Patienten strömt. Die Messeinheit 21A misst die Dichte des Bluts in der venösen Blutleitung 7 stromab der venösen Zugabestelle 20, an der während der Substitution Substitutionsflüssigkeit in die venöse Blutleitung 7 strömt.

Die venöse Messeinheit 21A verfügt über einen Ultraschallsender 21A' und einen Ultraschallempfänger 21A", die entlang einer Messstrecke angeordnet sind. Die Messstrecke kann beispielsweise durch eine nicht dargestellte venöse Tropfkammer oder durch einen sich an die Tropfkammer anschließenden Abschnitt der venösen Blutleitung verlaufen. Derartige Ultraschallmesseinrichtungen zur Messung der Dichte von Medien sind dem Fachmann bekannt. Die Messeinrichtungen beruhen auf der Messung der Ausbreitungsgeschwindigkeit von Ultraschallwellen, die von dem Sender 21A' ausgesandt und von dem Empfänger 21A" empfangen werden. Alternativ kann zur Messung des Bluts anstelle einer Ultraschallmesseinrichtung eine Messeinheit zum Messen der Dämpfung von Licht eingesetzt werden, die anstelle des Ultraschall-Senders- bzw. Empfängers über eine auf einer Seite der Messstrecke angeordnete Lichtquelle und einen auf der anderen Seite der Messstrecke angeordneten Lichtsensor verfügt.

Die Vorrichtung zur Detektion von Prä- und Postdilution weist weiterhin eine Auswerteinheit 22 auf, die über eine Datenleitung 23 mit der zentralen Steuer- und Recheneinheit 18 verbunden ist. Die Auswerteinheit 22 empfängt über eine weitere Datenleitung 24 die Messwerte der Messeinheit 21A.

Nachfolgend wird der Aufbau und die Funktionsweise der Vorrichtung zur Detektion einer Prä- und Postdilution im Einzelnen erläutert.

Während der extrakorporalen Blutbehandlung steuert die zentrale Steuer- und Recheneinheit 18 die Blutpumpe 8 derart an, dass Blut in die Blutkammer 3 des Dialysators mit der Blutflussrate Q_{b} strömt, und steuert die erste und zweite Dialysierflüssigkeitspumpe 13, 14 derart an, dass Dialysierflüssigkeit mit der Dialysierflüssigkeitsrate Q_{di} in die Dialysierflüssigkeitskammer 4 strömt und Dialysierflüssigkeit mit der Dialysierflüssigkeitsrate Q_{do} aus der Dialysierflüssigkeitskammer 4 strömt. Die Substituatpumpe 16 steuert die Steuereinheit 18 derart an, dass Substitutionsflüssigkeit mit der Substitutionsrate Qₛ dem Blut wahlweise stromauf und/oder stromab der Blutkammer zugeführt wird.

Zur Überwachung der Prä- oder Postdilution steuert die Steuereinheit 18 die Substituatpumpe 16 derart an, dass deren Förderrate vorzugsweise nur für ein vorgegebenes Zeitintervall um einen vorgegebenen Betrag verringert oder die Substituatpumpe 16 angehalten wird. Gleichzeitig steuert die Steuereinheit 18 die erste und die zweite Dialysierflüssigkeitspumpe 13 und 14 derart an, dass die Flussrate Q_{M}, mit der dem Blut Flüssigkeit über die Membran 2 des Dialysators oder Filters entzogen wird, wobei Q_{M} = Q_{do}-Q_{di} ist, gleichzeitig innerhalb des gleichen Zeitintervalls um den gleichen Betrag verringert wird, wie die Substitutionsrate verringert worden ist. Dies führt dazu, dass dem Blut über die Membran 2 des Dialysators 1 weniger Flüssigkeit (Ultrafiltrat) entzogen wird. Vor und nach dem Ändern der Förderraten bzw. Anhalten der beteiligten Pumpen misst die Messeinheit 21A die Dichte des Bluts oder des Blutinhaltsstoffes stromab der venösen Zugabestelle 20.

Es ist auch möglich, die Substitutionsrate Q_{S} und die Flussrate Q_{M}, mit der dem Blut Flüssigkeit über die Membran des Dialysators oder Filters entzogen wird, auf einen Wert von null einzustellen. Dies kann beispielsweise dadurch erreicht werden, dass der Dialysator oder Filter in einen Bypass-Betrieb geschaltet wird, so dass dann auch Q_{di} gleich null ist. Falls es zuvor eine Netto-Ultrafiltrationsrate gegeben hat, die einen Beitrag zu Q_{M} geleistet hat, werden in diesem Fall die Flussraten Q_{S} und Q_{M} nicht um den gleichen Betrag verringert, da Q_{M} um den Nettoultrafiltrationsbetrag größer war.

Die Auswerteinheit 22 weist eine Vergleichseinrichtung 22A auf, die den vor der Änderung der Förderarten der Pumpen gemessenen Wert für die Dichte des Bluts oder des Blutinhaltsstoffes mit dem unmittelbar nach der Änderung der Förderraten gemessenen Wert für die Dichte vergleicht. Die Messung der Dichte erfolgt innerhalb eines bestimmten Zeitintervalls nach der Änderung der Flussraten, da sich nach Ablauf des Zeitintervalls wieder die ursprünglichen Werte einstellen. Das Zeitintervall sollte auf jeden Fall kürzer als die Länge der Dichteänderung (Rechteckfunktion) sein, wobei auf empirische Werte zurückgegriffen werden kann. Dabei ist zu beachten, dass die Flussratenänderungen in den genannten Beispielen - Q_{S} und Q_{M} ändern sich um den gleichen Betrag - nur zu einer zeitliche begrenzten Änderung der Dichte führen. Auf der Grundlage der Veränderung der Dichte erkennt die Auswerteinheit dann, ob eine Dilution erfolgt und stellt fest, ob eine Prädilution oder Postdilution vorliegt.

Die von der Auswerteinheit 22 festgestellten Betriebszustände werden auf einer Anzeigeeinheit 25 angezeigt, die über eine Datenleitung 26 mit der Auswerteinheit 22 verbunden ist. Weiterhin erzeugt die Auswerteinheit zwei Steuersignale, die einerseits den Betriebszustand der Prädilution und andererseits den Betriebszustand der Postdilution signalisieren. Beide Steuersignale empfängt die Steuereinheit 22 über die Datenleitung 23, die in Abhängigkeit von dem jeweiligen Betriebszustand der Prä- oder Postdilution einen Eingriff in die Maschinensteuerung vornehmen kann.

Für den Fall der Postdilution stellt die Auswerteinheit 22 einen kurzzeitigen Anstieg der Dichte des Bluts an der Messstelle fest. Dies ist darauf zurückzuführen, dass sich das Blut nach dem Durchtritt durch die Blutkammer 3 des Dialysators 1 verdickt hat, da dem Blut über die Membran 2 des Dialysator 1 Flüssigkeit (Ultrafiltrat) entzogen worden ist. Da das bereits verdickte Blut bei der Postdilution nicht mehr ausreichend mit Substitutionsflüssigkeit verdünnt wird, steigt die Dichte des Bluts oder des Blutinhaltsstoffes stromab des Dialysators für einen bestimmten Zeitraum an. Für die Messung brauchen die Förderarten nur kurzzeitig geändert zu werden, d.h. nach erfolgter Messung können die ursprünglichen Förderraten wieder eingestellt werden, wodurch es zu einem entgegengesetzten- zeitlich wiederum begrenzten- Verhalten der Dichteänderung kommt.

Für den Fall einer Prädilution hingegen wird das in die Blutkammer 3 strömende Blut durch den Zufluss von Substitutionsflüssigkeit stromauf der Blutkammer verdünnt. Unmittelbar nach dem Zeitpunkt, zu dem die Förderraten der Pumpen verringert werden, gelangt zunächst noch verdünntes Blut in die Blutkammer, dem aber nach der Verringerung der Förderraten nicht mehr ausreichend Flüssigkeit über die Dialysatormembran entzogen wird. Folglich nimmt die Dichte des aus der Blutkammer austretenden und zum Patienten zurückströmenden Bluts kurzzeitig ab. Die Abnahme der Dichte wird wieder mit der Messeinheit 21A gemessen, wobei die Auswerteinheit 22 den Betriebszustand der Prädilution feststellt.

Die Vergleichseinrichtung 22 A der Auswerteinheit 22 bildet die Differenz zwischen den beiden Messwerten der Dichte vor und unmittelbar nach der Veränderung der Förderraten. Wenn der Betrag der Differenz größer als ein vorgegebener Grenzwert ist, d.h. sich die vor bzw. nach der Veränderung der Substitutionsrate gemessenen Werte deutlich voneinander unterscheiden, stellt die Auswerteinheit 22 fest, dass eine Dilution erfolgt. Darüber hinaus stellt die Auswerteinheit fest, ob eine Zunahme oder Abnahme der Dichte erfolgt, d.h. ob die Differenz der Messwerte positiv oder negativ ist.

Für den Fall einer Zunahme der Dichte um einen Betrag, der größer als ein vorgegebener Grenzwert ist, stellt die Auswerteinheit dann den Betriebszustand einer Postdilution fest. Wenn die Dichte um einen Betrag abgenommen hat, dessen Betrag größer als ein vorgegebener Grenzwert ist, stellt die Auswerteinheit dann den Betriebszustand einer Prädilution fest.

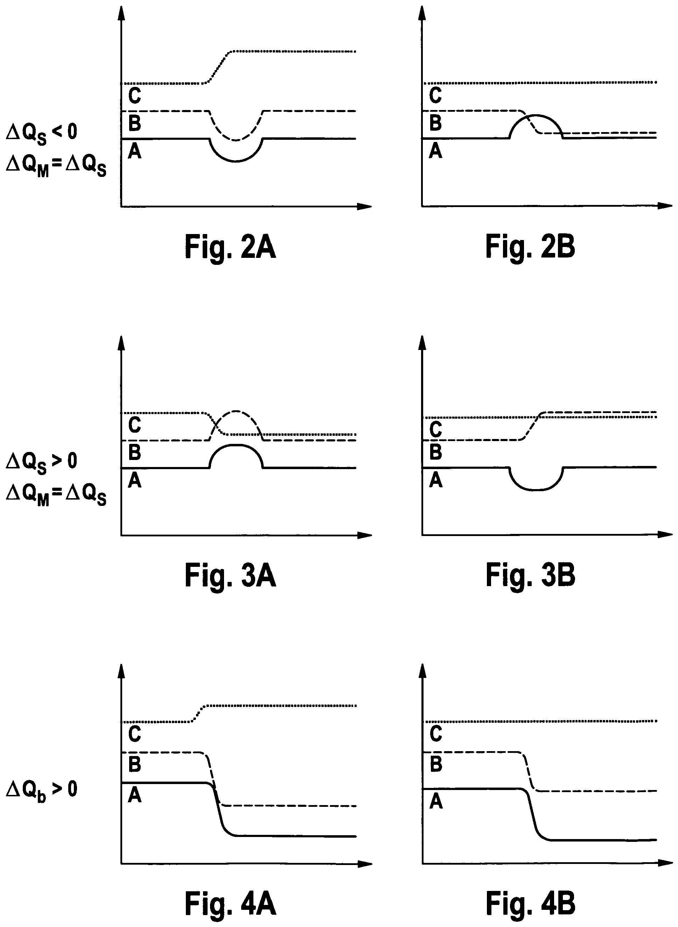

Die Figuren 2A und 2B zeigen den zeitlichen Verlauf der Dichte des Bluts für den Fall der Prädilution (Fig. 2A) und Postdilution (Fig. 2B), wobei einerseits die Substitutionsrate Qₛ um einen vorgegebenen Betrag ΔQₛ < 0 verringert und gleichzeitig die Flussrate Q_{M} mit der dem Blut Flüssigkeit entzogen wird, um den gleichen Betrag verringert wird.

Die mit A bezeichneten Grafen der Figuren 2A und 2B zeigen den zeitlichen Verlauf der Dichte für den Fall der Prä- oder Postdilution, wenn die Dichteänderung stromab der venösen Zugabestelle 20 mit der Messeinheit 21A gemessen wird, wie unter Bezugnahme auf Fig. 1 beschrieben ist.

Alternative Ausführungsformen sehen aber auch eine Messung der Dichteänderung stromauf der venösen Zugabestelle 20 und stromab der Blutkammer 3 oder stromab der arteriellen Zugabestelle 19 und stromauf der Blutkammer 3 des Dialysators 1 vor. Hierfür sind zwei weitere alternative Messeinheiten vorgesehen, die in Fig.1 mit 21 B und 21C bezeichnet sind. Die Messeinheit 21B misst die Dichte stromauf der venösen Zugabestelle 20 und stromab der Blutkammer 3, während die Messeinheit 21C die Dichte stromab der arteriellen Zugabestelle 19 und stromauf der Blutkammer 3 misst.

Die mit B bezeichneten Grafen der Figuren 2A und 2B zeigen den zeitlichen Verlauf der Dichte für den Fall der Prä- (Fig. 2A) oder Postdilution (Fig. 2B), wenn die Dichteänderung mit der Messeinheit 21B gemessen wird, während die Grafen C den zeitlichen Verlauf der Dichteänderung zeigen, wenn die Dichte mit der Messeinheit 21C gemessen wird.

Es zeigt sich, dass eine Variation der Substitutionsrate Q_{S} bei gleichzeitiger Änderung von Q_{M} auch zu einer Änderung der Dichte des Bluts stromauf der venösen Zugabestelle 20 und stromab der Blutkammer 3 führt. Die Dichte des Bluts nimmt sowohl bei der Prä- als auch Postdilution ab, wobei sich bei der Prädilution im Gegensatz zur Postdilution wieder der ursprüngliche Wert für die Dichte einstellt.

Weiterhin zeigt sich, dass eine Variation der Substitutionsrate Q_{S} auch zu einer Änderung der Dichte des Bluts stromab der arteriellen Zugabestelle 19 und stromauf der Blutkammer 3 führt. Die Dichte des Bluts nimmt bei der Prädilution zu, während sie bei der Postdilution weder zu noch abnimmt, d.h. gleich bleibt.

Alternative Ausführungsformen der Erfindung sehen eine Messung der Dichteänderung mit den Messeinheiten 21B oder 21C vor, wobei die Auswerteinheit auf eine Prä- oder Postdilution auf der Grundlage der Art der Dichteänderung schließt, die in den Figuren 2A und 2B gezeigt ist. Dem Fachmann sind geeignete Einrichtungen bekannt, mit denen die Signale ausgewertet werden könne. Diese Einrichtungen können über Komparatoren, Zeitglieder etc. verfügen.

Es ist auch möglich die oben angegebenen Messverfahren miteinander zu kombinieren, so dass auf der Grundlage von zwei oder drei Messungen an unterschiedlichen Messstellen eine Prä- oder Postdilution erkannt werden kann. Beispielsweise kann auf eine Prä- bzw. Postdilution geschlossen werden, wenn mindestens bei zwei Messungen an unterschiedlichen Messstellen eine für eine Prä- oder Postdilution charakteristische Veränderung der Signale erkannt wird.

Die Figuren 3A (Prädilution) und 3B (Postdilution) zeigen den zeitlichen Verlauf der Dichte des Bluts oder eines Blutinhaltsstoffes, der mit den Messeinheiten 21A, 21B und 21C gemessen wird, wenn einerseits die Substitutionsrate Q_{S} um einen vorgegebenen Betrag vergrößert und gleichzeitig die Flussrate, mit der dem Blut Flüssigkeit über die Membran 2 entzogen wird, um den gleichen Betrag vergrößert wird. Die Grafen sind analog zu den Fig. 2A und 2B wieder mit A, B und C bezeichnet.

Es zeigt sich, dass eine Erhöhung der Substitutionsrate Q_{S} bei gleichzeitiger Änderung von Q_{M} zu einer Änderung der Dichte an allen drei Messstellen im Falle der Prädilution führt. Eine Erhöhung von Q_{S} und Q_{M} hat stromab der venösen Zugabestelle 20 bei einer Prädilution im Gegensatz zu einer Verringerung der Rate nicht eine Abnahme, sondern eine Zunahme der Dichte zur Folge und führt bei einer Postdilution nicht zu einer Erhöhung, sondern Verringerung der Dichte (Graf A). Stromauf der venösen Zugabestelle 20 und stromab der Blutkammer 3 nimmt die Dichte sowohl bei der Prä- als auch Postdilution zu, wobei sich bei der Prädilution im Gegensatz zur Postdilution wieder der ursprüngliche Wert für die Dichte einstellt (Graf B). Stromab der arteriellen Zugabestelle 19 und stromauf der Blutkammer 3 nimmt die Dichte bei der Prädilution ab, während sie bei der Postdilution weder zu noch abnimmt, d.h. gleich bleibt (Graf C).

Bei einer alternativen Ausführungsform sind Steuereinheit 18 und Auswerteinheit 22 derart ausgebildet, dass die Substitutionsrate Q_{S} und die Flussrate Q_{M} verringert werden und auf der Grundlage der Veränderung der Dichte auf eine Prä- oder Postdilution geschlossen wird, wie unter Bezugnahme auf die Figuren 3A und 3B beschrieben ist.

Bei einem weiteren Ausführungsbeispiel wird nicht die Substitutionsrate Q_{S} oder die Flussrate Q_{M}, sondern die Blutflussrate Q_{b} verändert (Figuren 4A und 4B). Die Steuereinheit 18 steuert die Blutpumpe 8 bei dieser Ausführungsform derart an, dass die Blutflussrate Q_{b} um einen vorgegebenen Betrag erhöht AQ_{b} wird. Die Grafen A, B, C zeigen wieder den zeitlichen Verlauf der Dichte des Bluts oder des Blutinhaltsstoffes, die mit den drei Messeinheiten 21A, 21B und 21C gemessen wird. Es zeigt sich, dass mit den Messeinheiten 21A und 21B eine Prä- oder Postdilution nur unter genauer quantitativer Auswertung der Dichteänderung nachgewiesen werden kann. Daher wertet die Auswerteinheit bevorzugt die Messwerte der Messeinheit 21 C aus, mit der die Dichte stromab der arteriellen Zugabestelle 19 und stromauf der Blutkammer 3 des Dialysators gemessen wird. Die Auswerteinheit 22 stellt eine Prädilution fest, wenn die Dichte um einen vorgegebenen Betrag zugenommen hat und stellt eine Postdilution fest, wenn die Dichte nicht um einen vorgegebenen Betrag zugenommen hat, d.h. gleich geblieben ist. Es ist natürlich auch denkbar, dass die Blutflussrate Q_{B} um einen vorgegebenen Betrag verringert wird. Dann verlaufen die Messungen jeweils in die entgegengesetzte Richtung.

## Patentansprüche

1. Vorrichtung zur Überwachung der Zufuhr von Substitutionsflüssigkeit für eine Vorrichtung zur extrakorporalen Blutbehandlung, wobei die extrakorporale Blutbehandlungsvorrichtung aufweist:
einen extrakorporalen Blutkreislauf (5A), der eine erste Kammer (3) eines durch eine Membran (2) in die erste Kammer und eine zweite Kammer (4) unterteilten Dialysators (1) oder Filters einschließt, und ein Flüssigkeitssystem (5B), das die zweite Kammer des Dialysators oder Filters einschließt,
eine Einrichtung (8) zum Zuführen von Blut in die erste Kammer des Dialysators oder Filters mit einer bestimmten Blutflussrate Q_{B},
eine Substitutionseinrichtung (16) zum Zuführen von Substitutionsflüssigkeit dem Blut stromauf oder stromab des Dialysators oder Filters mit einer bestimmten Substitutionsrate Q_{S} und
eine Ultrafiltrationseinrichtung (13, 14) zum Entziehen von Flüssigkeit dem Blut über die Membran des Dialysators oder Filters mit einer bestimmten Flussrate Q_{M},
**dadurch gekennzeichnet, dass**
die Vorrichtung zur Überwachung der Zufuhr von Substitutionsflüssigkeit aufweist:
eine Messeinheit (21) zum Messen der Dichte des Bluts oder eines Blutinhaltsstoffes im Blutkreislauf des Dialysators oder Filters,
eine Steuereinheit (18) zum Steuern der Substitutionseinrichtung (16) und/oder der Einrichtung (8) zum Zuführen von Blut und/oder der Ultrafiltrationseinrichtung (13, 14), wobei die Steuereinheit (18) derart ausgebildet ist, dass die Substitutionsrate Q_{S} und/oder die Blutflussrate Q_{B} und/oder die Flussrate Q_{M} der durch die Membran des Dialysators oder Filters entzogenen Flüssigkeit verändert wird, und
eine Auswerteinheit (22), die eine Vergleichseinrichtung (22A) aufweist, die die Differenz zwischen dem vor der Änderung der Flussrate gemessenen Wert für die Dichte des Bluts oder eines Blutinhaltsstoffes und dem innerhalb eines bestimmten Zeitintervalls nach der Änderung der Flussrate gemessenen Wert für die Dichte des Bluts oder eines Blutinhaltsstoffes bildet und mit einem vorgegebenen Grenzwert vergleicht, um eine Zu- oder Abnahme der Dichte des Bluts oder eines Blutinhaltsstoffes um einen vorgegebenen Betrag feststellen zu können,
wobei die Auswerteinheit (22) derart ausgebildet ist, dass auf der Grundlage des Betrages und/oder der Richtung der Veränderung der Dichte des Bluts oder eines Blutinhaltsstoffes darauf geschlossen wird, ob eine Dilution erfolgt und ob eine Prädilution oder Postdilution vorliegt.

2. Vorrichtung nach Anspruch 1, **dadurch gekennzeichnet, dass** die Steuereinheit (18) zum Steuern der Substitutionseinrichtung derart ausgebildet ist, dass für die Detektion der Zufuhr von Substitutionsflüssigkeit stromauf oder stromab des Dialysators oder Filters die Substitutionsrate Q_{S} um einen vorgegebenen Betrag verringert wird, während die Flussrate Q_{M} der durch die Membran des Dialysators oder Filters entzogenen Flüssigkeit verringert wird oder die Substitutionsrate Q_{S} um einen vorgegebenen Betrag vergrößert wird, während die Flussrate Q_{M} der durch die Membran des Dialysators oder Filters entzogenen Flüssigkeit vergrößert wird.

3. Vorrichtung nach Anspruch 2, **dadurch gekennzeichnet, dass** die Steuereinheit (18) zum Steuern der Substitutionseinrichtung derart ausgebildet ist, dass für die Detektion der Zufuhr von Substitutionsflüssigkeit stromauf oder stromab des Dialysators oder Filters die Substitutionsrate Q_{S} um den gleichen Betrag verringert wird, wie die Flussrate Q_{M} der durch die Membran des Dialysators oder Filters entzogenen Flüssigkeit verringert wird oder die Substitutionsrate Q_{S} um den gleichen Betrag vergrößert wird, wie die Flussrate Q_{M} der durch die Membran des Dialysators oder Filters entzogenen Flüssigkeit vergrößert wird.

4. Vorrichtung nach einem der Ansprüche 1 bis 3, **dadurch gekennzeichnet**, die Steuereinheit (18) zum Steuern der Substitutionseinrichtung derart ausgebildet ist, dass nach der Verringerung bzw. Vergrößerung der Substitutionsrate Q_{S} und/oder der Blutflussrate Q_{B} und/oder der Flussrate Q_{M} der durch die Membran des Dialysators oder Filters entzogenen Flüssigkeit um einen vorgegebenen Betrag für ein vorgegebenes Zeitintervall nach Ablauf des vorgegebenen Zeitintervalls die Substitutionsrate Q_{S} und/oder die Blutflussrate Q_{B} und/oder die Flussrate Q_{M} der durch die Membran des Dialysators oder Filters entzogenen Flüssigkeit vergrößert bzw. verringert wird.

5. Vorrichtung nach einem der Ansprüche 1 bis 4, **dadurch gekennzeichnet, dass** die Messeinheit (21A, 21B, 21C) eine Messeinheit zum Messen der Dichte des Bluts oder des Blutinhaltsstoffes im Blutkreislauf stromab der Stelle des Blutkreislaufs ist, an der dem Blutkreislauf bei einer Prädilution Substitutionsflüssigkeit zugeführt wird.

6. Vorrichtung nach Anspruch 5, **dadurch gekennzeichnet, dass** die Messeinheit (21C) eine Messeinheit zum Messen der Dichte des Bluts stromab der Stelle des Blutkreislauf, an der dem Blutkreislauf bei einer Prädilution Substitutionsflüssigkeit zugeführt wird, und stromauf der ersten Kammer (3) des Dialysators (1) ist.

7. Vorrichtung nach einem der Ansprüche 1 bis 5, **dadurch gekennzeichnet, dass** die Messeinheit (21B) eine Messeinheit zum Messen der Dichte des Bluts oder des Blutinhaltsstoffes stromab der ersten Kammer (3) des Dialysators (1) oder Filters und stromauf der Stelle des Blutkreislaufs ist, an der dem Blutkreislauf bei einer Postdilution Substitutionsflüssigkeit zugeführt wird.

8. Vorrichtung nach einem der Ansprüche 1 bis 5, **dadurch gekennzeichnet, dass** die Messeinheit (21A) eine Messeinheit zum Messen der Dichte des Bluts oder des Blutinhaltsstoffes stromab der Stelle des Blutkreislaufs ist, an der dem Blutkreislauf bei einer Postdilution Substitutionsflüssigkeit zugeführt wird.

9. Vorrichtung nach einem der Ansprüche 1 bis 8, **dadurch gekennzeichnet, dass** die Messeinheit (21) eine Ultraschallmesseinrichtung ist, die einen auf einer Seite einer Messtrecke angeordneten Ultraschallsender (21') und einen auf der anderen Seite der Messtrecke angeordneten Ultraschallempfänger (21") für die Messung der Änderung der Ausbreitungsgeschwindigkeit von Ultraschall im Blut entlang der Messtrecke aufweist.

10. Vorrichtung nach einem der Ansprüche 1 bis 8, **dadurch gekennzeichnet, dass** die Messeinheit (21) eine optische Messeinrichtung ist, die eine auf einer Seite einer Messtrecke angeordnete Lichtquelle (21') und einen auf der anderen Seite der Messtrecke angeordnete Lichtsensor (21") für die Messung der Dämpfung von Licht im Blut entlang der Messtrecke aufweist.

11. Vorrichtung nach einem der Ansprüche 1 bis 10, **dadurch gekennzeichnet, dass** die Steuereinheit (22) derart mit der Auswerteinheit (22) zusammenwirkt, dass die Steuereineinheit ein den Betriebzustand der Prädilution signalisierendes Signal erzeugt, wenn auf der Grundlage der Änderung der Dichte des Bluts oder des Blutinhaltsstoffes auf eine Zufuhr von Substitutionsflüssigkeit stromauf des Dialysators oder Filters geschlossen wird, und/oder ein den Betriebzustand der Postdilution signalisierendes Signal erzeugt, wenn auf der Grundlage der Änderung der Dichte des Bluts oder des Blutinhaltsstoffes auf eine Zufuhr von Substitutionsflüssigkeit stromab des Dialysators oder Filters geschlossen wird.

12. Blutbehandlungsvorrichtung mit einer Vorrichtung zur Detektion der Zufuhr von Substitutionsflüssigkeit nach einem der Ansprüche 1 bis 11.

13. Blutbehandlungsvorrichtung nach Anspruch 12, **dadurch gekennzeichnet, dass** die Blutbehandlungsvorrichtung aufweist:
einen extrakorporalen Blutkreislauf (5A), der eine zu einer ersten Kammer (3) eines durch eine Membran (2) in die erste Kammer (3) und eine zweite Kammer (4) unterteilten Dialysators (1) oder Filters führende Blutzuführleitung (6) und eine von der ersten Kammer des Dialysators oder Filters abgehende Blutabführleitung (7) sowie den Dialysator oder Filter aufweist,
ein Flüssigkeitssystem (5B), das mit der zweiten Kammer (4) des Dialysators (1) oder Filters verbunden ist,
eine Substitutionseinrichtung (19) mit einer zu einer ersten Zugabestelle (19) an der Blutzuführleitung (6) führenden ersten Substituatleitung (15, 15a) zum Zuführen von Substitutionsflüssigkeit stromauf des Dialysators (1) oder Filters und einer zu einer zweiten Zugabestelle (20) an der Blutabführleitung (7) führenden zweiten Substituatleitung (15, 15b) zum Zuführen von Substitutionsflüssigkeit stromab des Dialysators oder Filters,
eine Ultrafiltrationseinrichtung (18), mit der dem Blut Flüssigkeit über die Membran (2) des Dialysators (1) oder Filters entzogen werden kann.

## Claims

1. Device for monitoring the supply of substitution fluid for an apparatus for extracorporeal blood treatment, wherein the extracorporeal blood treatment apparatus comprises:
an extracorporeal blood circuit (5A), which comprises a first chamber (3) of a dialyser (1) or filter divided by a membrane (2) into the first chamber and a second chamber (4), and a fluid system (5B), which comprises the second chamber of the dialyser or filter,
a device (8) for supplying blood to the first chamber of the dialyser or filter at a specific blood flow rate Q_{B},
a substitution device (16) for supplying substitution fluid to the blood upstream or
downstream of the dialyser or filter at a specific substitution rate Q_{S} and
an ultrafiltration device (13, 14) for withdrawing fluid from the blood via the membrane of the dialyser or filter at a specific flow rate Q_{M},
**characterised in that**
the device for monitoring the supply of substitution fluid comprises:
a measuring unit (21) for measuring the density of the blood or a blood constituent in the blood circuit of the dialyser or filter,
a control unit (18) for controlling the substitution device (16) and/or the device (8) for supplying blood and/or the ultrafiltration device (13, 14), wherein the control unit (18) is designed in such a way that substitution rate Q_{S} and/or blood flow rate Q_{B} and/or flow rate Q_{M} of the fluid withdrawn through the membrane of the dialyser or filter is changed, and
an evaluation unit (22) which comprises a comparison device (22A) that generates the difference between the value for the density of the blood or a blood constituent measured before the change in the delivery rate and the value for the density of the blood or a blood constituent measured within a specific time interval after the change in the flow rate and compares said difference with a preset threshold value in order to be able to ascertain an increase or decrease in the density of the blood or a blood constituent by a preset amount,
wherein the evaluation unit (22) is designed such that it is concluded, on the basis of the amount and/or the direction of the change in density of the blood or a blood constituent, whether a dilution is taking place and whether a pre-dilution or post-dilution is present.

2. The device according to claim 1, **characterised in that** the control unit (18) for controlling the substitution device is designed in such a way that, for the detection of the supply of substitution fluid upstream or downstream of the dialyser or filter, substitution rate Qs is reduced by a preset amount, whilst flow rate Q_{M} of the fluid withdrawn through the membrane of the dialyser or filter is reduced, or substitution rate Q_{S} is increased by a preset amount, whilst flow rate Q_{M} of the fluid withdrawn through the membrane of the dialyser or filter is increased.

3. The device according to claim 2, **characterised in that** the control unit (18) for controlling the substitution device is designed in such a way that, for the detection of the supply of substitution fluid upstream or downstream of the dialyser or filter, substitution rate Q_{S} is reduced by the same amount as flow rate Q_{M} of the fluid withdrawn through the membrane of the dialyser or filter is reduced, or substitution rate Q_{S} is increased by the same amount as flow rate Q_{M} of the fluid withdrawn through the membrane of the dialyser or filter is increased.

4. The device according to any one of claims 1 to 3, **characterised in that** the control device (18) for controlling the substitution device is designed in such a way that, after the reduction or increase in substitution rate Q_{S} and/or blood flow rate Q_{B} and/or flow rate Q_{M} of the fluid withdrawn through the membrane of the dialyser or filter by a preset amount for a preset time interval, substitution rate Qs and/or blood flow rate Q_{B} and/or flow rate Q_{M} of the fluid withdrawn through the membrane of the dialyser or filter is increased or reduced after the lapse of the preset time interval.

5. The device according to any one of claims 1 to 4, **characterised in that** the measuring unit (21A, 21B, 21C) is a measuring unit for measuring the density of the blood or the blood constituent in the blood circuit downstream of the point of the blood circuit at which substitution fluid is fed to the blood circuit in the case of a pre-dilution.

6. The device according to claim 5, **characterised in that** the measuring unit (21C) is a measuring unit for measuring the density of the blood downstream of the point of the blood circuit at which substitution fluid is fed to the blood circuit in a pre-dilution, and upstream of the first chamber (3) of the dialyser (1).

7. The device according to any one of claims 1 to 5, **characterised in that** the measuring unit (21B) is a measuring unit for measuring the density of the blood or the blood constituent downstream of the first chamber (3) of the dialyser (1) or filter and upstream of the point of the blood circuit at which substitution fluid is fed to the blood circuit in the case of a post-dilution.

8. The device according to any one of claims 1 to 5, **characterised in that** the measuring unit (21 A) is a measuring unit for measuring the density of the blood or the blood constituent downstream of the point of the blood circuit at which substitution fluid is fed to the blood circuit in the case of a post-dilution.

9. The device according to any one of claims 1 to 8, **characterised in that** the measuring unit (21) is an ultrasound measuring device, which comprises an ultrasound transmitter (21') disposed on one side of a measuring distance and an ultrasound receiver (21") disposed on the other side of the measuring distance for the measurement of the change in the propagation speed of ultrasound in the blood along the measuring distance.

10. The device according to any one of claims 1 to 8, **characterised in that** the measuring unit (21) is an optical measuring device, which comprises a light source (21') disposed on one side of a measuring distance and a light sensor (21") disposed on the other side of the measuring distance for the measurement of the attenuation of light in the blood along the measuring distance.

11. The device according to any one of claims 1 to 10, **characterised in that** the control unit (22) cooperates with the evaluation unit (22) in such a way that the control unit generates a signal signalling the operational state of pre-dilution when it is concluded that there is a supply of substitution fluid upstream of the dialyser or filter on the basis of the change in the density of the blood or the blood constituent, and/or generates a signal signalling the operational state of post-dilution when it is concluded that there is a supply of substitution fluid downstream of the dialyser or filter on the basis of the change in the density of the blood or the blood constituent.

12. A blood treatment apparatus with a device for detecting the supply of substitution fluid according to any one of claims 1 to 11.

13. The blood treatment apparatus according to claim 12, **characterised in that** the blood treatment apparatus comprises:
an extracorporeal blood circuit (5A), which comprises a blood supply line (6) leading to a first chamber (3) of a dialyser (1) or filter divided by a membrane (2) into the first chamber (3) and a second chamber (4) and a blood discharge line (7) leading away from the first chamber of the dialyser or filter as well as the dialyser or filter,
a fluid system (5B), which is connected to the second chamber (4) of the dialyser (1) or filter,
a substitution device (19) with a first substituate line (15, 15a) leading to a first admission point (19) on that the blood supply line (6) for supplying substitution fluid upstream of the dialyser (1) or filter and a second substituate line (15, 15b) leading to a second admission point (20) on the blood discharge line (7) for supplying substitution fluid downstream of the dialyser or filter,
an ultrafiltration device (18), with which fluid can be withdrawn from the blood via the membrane (2) of the dialyser (1) or filter.

## Revendications

1. Dispositif de surveillance de l'amenée de liquide de substitution pour un dispositif de traitement extracorporel du sang, dans lequel le dispositif de traitement extracorporel du sang présente :
une circulation sanguine extracorporelle (5A) qui comprend une première chambre (3) d'un dialyseur (1) ou filtre subdivisé par une membrane (2) dans la première chambre et
une deuxième chambre (4), et un système de liquide (5B) qui comprend la deuxième chambre du dialyseur ou filtre,
un système (8) pour amener du sang dans la première chambre du dialyseur ou filtre avec un débit sanguin Q_{B} déterminé,
un système de substitution (16) pour amener du fluide de substitution au sang en amont ou en aval du dialyseur ou filtre avec un taux de substitution Q_{S} déterminé et
un système d'ultrafiltration (13, 14) pour retirer du liquide du sang par la membrane du dialyseur ou filtre avec un débit Q_{M} déterminé,
**caractérisé en ce que**
le dispositif de surveillance de l'amenée de liquide de substitution présente :
une unité de mesure (21) pour mesurer la densité du sang ou d'un composant sanguin dans la circulation sanguine du dialyseur ou filtre,
une unité de commande (18) pour commander le système de substitution (16) et/ou le système (8) pour amener le sang et/ou le système d'ultrafiltration (13, 14), dans lequel l'unité de commande (18) est conçue de telle sorte que le taux de substitution Q_{S} et/ou le débit sanguin Q_{B} et/ou le débit Q_{M} du liquide retiré par la membrane du dialyseur ou filtre soit modifié, et
une unité d'analyse (22) qui présente un système de comparaison (22A) qui forme la différence entre la valeur mesurée avant la modification du débit pour la densité du sang ou d'un composant sanguin, et la valeur mesurée dans un intervalle temporel déterminé après la modification du débit pour la densité du sang ou d'un composant sanguin et la compare à une valeur seuil prédéterminée pour pouvoir relever une augmentation ou une diminution de la densité du sang ou d'un composant sanguin, d'un montant prédéterminé,
dans lequel l'unité d'analyse (22) est conçue de telle sorte que sur la base du montant et/ou de l'orientation de la modification de la densité du sang ou d'un composant sanguin, on conclut si une dilution s'effectue ou si une prédilution ou une post-dilution existe.

2. Dispositif selon la revendication 1, **caractérisé en ce que** l'unité de commande (18) pour commander le système de substitution est conçue de telle sorte que pour la détection de l'amenée de liquide de substitution en amont ou en aval du dialyseur ou filtre, le taux de substitution Q_{S} est diminué d'un montant prédéterminé, tandis que le débit Q_{M} du liquide retiré par la membrane du dialyseur ou filtre est diminué ou le taux de substitution Q_{S} est augmenté d'un montant prédéterminé, tandis que le débit Q_{M} du liquide retiré par la membrane du dialyseur ou filtre est augmenté.

3. Dispositif selon la revendication 2, **caractérisé en ce que** l'unité de commande (18) pour commander le système de substitution est conçue de telle sorte que pour la détection de l'amenée de liquide de substitution en amont ou en aval du dialyseur ou filtre, le taux de substitution Q_{S} est diminué du même montant que le débit Q_{M} du liquide retiré par la membrane du dialyseur ou filtre est diminué ou le taux de substitution Q_{S} est augmenté du même montant que le débit Q_{M} du liquide retiré par la membrane du dialyseur ou filtre est augmenté.

4. Dispositif selon l'une des revendications 1 à 3, **caractérisé en ce que** l'unité de commande (18) pour commander le système de substitution est conçue de telle sorte qu'après la diminution ou l'augmentation du taux de substitution Q_{S} et/ou du débit sanguin Q_{B} et/ou du débit Q_{M} du liquide retiré par la membrane du dialyseur ou filtre d'un montant prédéterminé pour un intervalle temporel prédéterminé après écoulement de l'intervalle temporel prédéterminé, le taux de substitution Q_{S} et/ou le débit sanguin Q_{B} et/ou le débit Q_{M} du liquide retiré par la membrane du dialyseur ou filtre est augmenté ou diminué.

5. Dispositif selon l'une des revendications 1 à 4, **caractérisé en ce que** l'unité de mesure (21A, 21B, 21C) est une unité de mesure pour mesurer la densité du sang ou du composant sanguin dans la circulation sanguine en aval du site de la circulation sanguine, au niveau duquel du liquide de substitution est amené à la circulation sanguine lors d'une prédilution.

6. Dispositif selon la revendication 5, **caractérisé en ce que** l'unité de mesure (21C) est une unité de mesure pour mesurer la densité du sang en aval du site de la circulation sanguine, au niveau duquel du liquide de substitution est amené à la circulation sanguine lors d'une prédilution et en amont de la première chambre (3) du dialyseur (1).

7. Dispositif selon l'une des revendications 1 à 5, **caractérisé en ce que** l'unité de mesure (21B) est une unité de mesure pour mesurer la densité du sang ou du composant sanguin en aval de la première chambre (3) du dialyseur (1) ou filtre et en amont du site de circulation sanguine, au niveau duquel du liquide de substitution est amené à la circulation sanguine lors d'une postdilution.

8. Dispositif selon l'une des revendications 1 à 5, **caractérisé en ce que** l'unité de mesure (21B) est une unité de mesure pour mesurer la densité du sang ou du composant sanguin en aval du site de circulation sanguine, au niveau duquel du liquide de substitution est amené à la circulation sanguine lors d'une postdilution.

9. Dispositif selon l'une des revendications 1 à 8, **caractérisé en ce que** l'unité de mesure (21) est un système de mesure à ultrasons qui présente un émetteur d'ultrasons (21') disposé d'un côté d'un parcours de mesure et un récepteur d'ultrasons (21") disposé de l'autre côté du parcours de mesure pour la mesure de la modification de la vitesse de propagation d'ultrasons dans le sang le long du parcours de mesure.

10. Dispositif selon l'une des revendications 1 à 8, **caractérisé en ce que** l'unité de mesure (21) est un système de mesure optique qui présente une source de lumière (21') disposée d'un côté d'un parcours de mesure et un capteur de lumière (21") disposé de l'autre côté du parcours de mesure pour la mesure de l'atténuation de la lumière dans le sang le long du parcours de mesure.

11. Dispositif selon l'une des revendications 1 à 10, **caractérisé en ce que** l'unité de commande (22) coopère avec l'unité d'analyse (22) de telle sorte que l'unité de commande génère un signal signalisant l'état opérationnel de la prédilution lorsque, sur la base de la modification de la densité du sang ou du composant sanguin, on conclut à une amenée de liquide de substitution en amont du dialyseur ou filtre et/ou génère un signal signalisant l'état opérationnel de la postdilution lorsque, sur la base de la modification de la densité du sang ou du composant sanguin, on conclut à une amenée de liquide de substitution en aval du dialyseur ou filtre.

12. Dispositif de traitement du sang avec un dispositif de détection de l'amenée de liquide de substitution selon l'une des revendications 1 à 11.

13. Dispositif de traitement du sang selon la revendication 12, **caractérisé en ce que** le dispositif de traitement du sang présente :
une circulation sanguine extracorporelle (5A) qui présente une conduite d'amenée de sang (6) conduisant à une première chambre (3) d'un dialyseur (1) ou filtre subdivisé par une membrane (2) en la première chambre (3) et une deuxième chambre (4) et une conduite d'évacuation du sang (7) partant de la première chambre du dialyseur ou filtre, ainsi que le dialyseur ou filtre,
un système de liquide (5B) qui est relié à la deuxième chambre (4) du dialyseur (1) ou filtre,
un système de substitution (19) avec une première conduite de substitut (15, 15a) conduisant à un premier site d'addition (19) au niveau de la conduite d'amenée de sang (6) pour amener du liquide de substitution en amont du dialyseur (1) ou filtre et une deuxième conduite de substitut (15, 15b) conduisant à un deuxième site d'addition (20) au niveau de la conduite d'évacuation de sang (7) pour amener du liquide de substitution en aval du dialyseur ou filtre,
un système d'ultrafiltration (18) avec lequel du liquide peut être retiré du sang par l'intermédiaire de la membrane (2) du dialyseur (1) ou filtre.
